# EUROPEAN PATENT APPLICATION

(11) **EP 4 400 637 A1**
(43) Date of publication of application: **17.07.2024**
(21) Application number: 22866732.5
(22) Date of filing: 08.09.2022
(51) Int. Cl.: C40B 50/06, C40B 40/06

(54) **METHOD FOR CONSTRUCTING CAPTURE LIBRARY HAVING HIGH TEST PERFORMANCE, AND KIT**

(30) Priority: 09.09.2021 CN 202111052998
(71) Applicant: Berry Genomics Co., Ltd, Beijing 102206 (CN)
(72) Inventor: CHEN, Di, Beijing 102206 (CN); LI, Dongning, Beijing 102206 (CN); ZHANG, Jianguang, Beijing 102206 (CN)
(74) Representative: Plasseraud IP
(86) International application number: PCT/CN2022/117953
(87) International publication number: WO 2023/036271

(57) **Abstract**

The present invention relates to a method for constructing a capture library suitable for next-generation sequencing platforms, comprising the following steps: 1) obtaining fragmented DNAs; (2) connecting the fragmented DNAs to a Y-shaped linker to obtain a pre-library; (3) hybridizing the pre-library with a hybridization probe to obtain a hybridization product; and (4) eluting the hybridization product to obtain a capture library. The present invention further relates to a kit for carrying out the above-mentioned method.

## Description

### FIELD OF THE DISCLOSURE

The present invention belongs to the field of molecular biology, specifically relates to a method and a kit for constructing a hybrid capture library.

### BACKGROUND OF THE DISCLOSURE

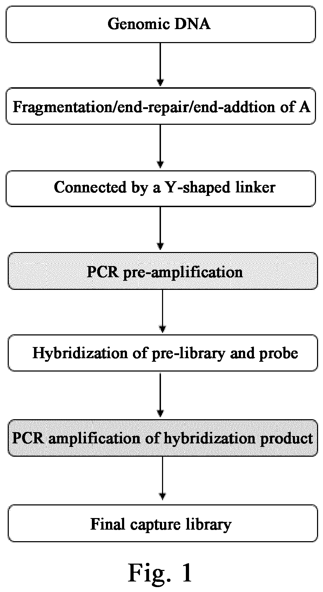

Exon capture is a technique that uses a probe to capture and enrich the DNA sequences of exon region, which is widely used in scientific research and clinical detection. Compared with whole genome sequencing, it has a lower cost, a shorter cycle, a better coverage, and is more economic and efficient. The construction of traditional exon capture library generally includes the following steps: performing a genomic DNA fragmentation, performing end-repair and end-addition of A, followed by ligation of a linker and a tag sequence, and obtaining a pre-library by a first round of PCR amplification; hybridizing the pre-library with a hybridization probe, and obtaining a final capture library by a second round of PCR amplification after purification (see **FIG. 1****:** flow diagram of traditional exon capture).

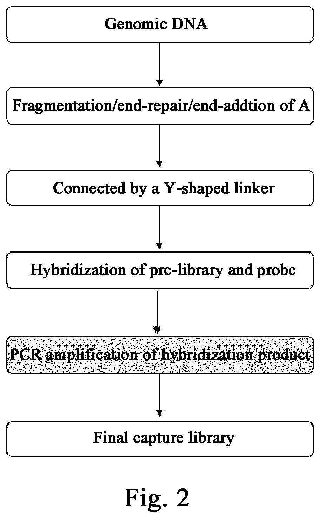

DNA enrichment by PCR is a common technology in the field of Next-generation sequencing (NGS). Although the application of PCR to exon capture achieves amplification of the capture product and obtains the library amount required for on-board sequencing, it also brings amplification errors and biases, making it impossible to perfectly present the original genome sequence. For example, the dominant amplification of some specific DNAs results in the inability to achieve a balanced amplification of DNAs, which in turn results in uneven coverage of the target area to the captured data, ultimately leading to detection errors or missed detections. The application of PCR-Free technology can perfectly circumvent the above shortcomings and eliminate steps such as amplification, streamlining the experimental process and reducing library construction costs. The traditional PCR-Free technology is more commonly used for the direct on-board sequencing of fragmented DNAs after connecting with a linker. Since PCR-Free technology requires a high input of DNA templates, and the probe capture process must take capture efficiency into consideration, it will cause waste of a large amount of DNA templates. At present, in the process of generally covering probe capture experimental methods, the initial DNA input amount needs to be as high as 500ng to 3µg, and PCR amplification is involved both before and after the probe capture step. Prior to the present invention, the inventors achieved technical optimization without PCR before capture under a low DNA initial input condition (see **FIG. 2****:** flow diagram of exon capture after optimization in this laboratory). However, the problem of still requiring the PCR amplification after hybridization capture remains unresolved.

Therefore, there is still a need to establish a PCR-Free hybridization capture process that is simple and economical, can avoid amplification errors and biases, achieve an improved detection performance and effective data utilization, and can implement a small amount of DNA input.

### SUMMARY DESCRIPTION

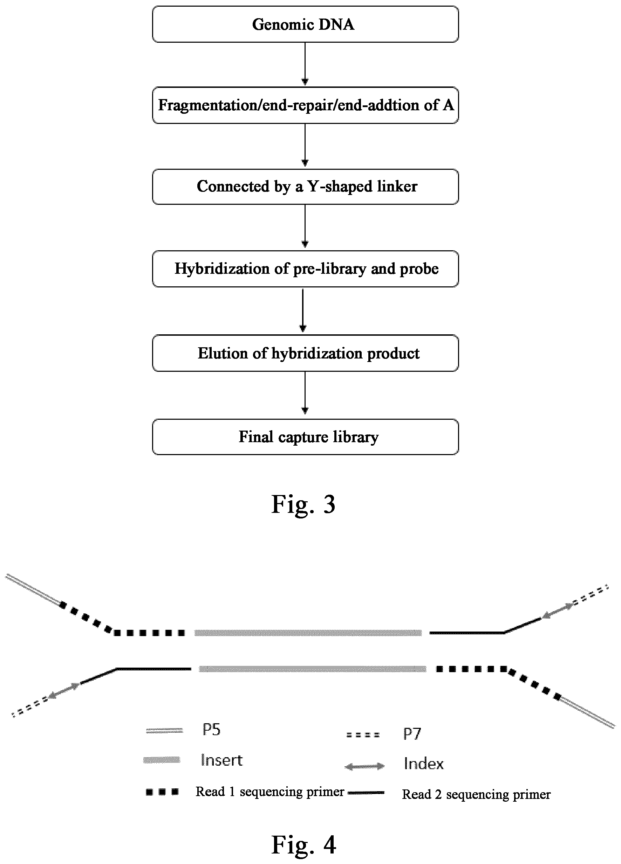

In view of a higher pursuit for detection performance of the captured data and the requirements for further cost saving and simplification of library construction process, and on the basis of the prior invention (described in the published patent application CN110409001A, which is incorporated herein by reference in its entirety), i.e. no PCR before capture, the inventors propose a method for constructing a capture library without PCR amplifications both before and after capture (see **FIG. 3****:** flow diagram of the PCR-Free library construction method of the entire library construction process of the present invention).

The present invention is based on the following facts discovered by the inventors:
(1) The detection performance of Indel is significantly improved by utilizing the original experimental process without PCR before capture and reducing the number of PCR cycles after capture. It is then occurred to the inventors that whether the optimal detection performance may be achieved if PCR can be completely removed.
(2) When initiated with the theoretically calculated DNAs of 50 to 100ng and without PCR before and after capture, the total amount of hybridization captured library is enough for on-board sequencing.
(3) The single-stranded DNAs obtained by hybridization capture of the pre-library connected with a long Y-shaped linker already have all the sequences required for on-board sequencing.
(4) After alkaline denaturation and neutralization reactions, the low-concentration double-stranded DNA library can be stably stored at -20°C for more than ten days, and the stability of the single-stranded DNA library can meet the requirements for on-board sequencing.
(5) Alkali denaturation can open the double-stranded DNAs between the probe and the hybridization product, the double-stranded DNAs between Cot-1 and the hybridization product, and can also open the connection between streptavidin and biotin. After alkaline denaturation treatment, a large number of biotin-labeled probes, Cot-1 and unsuccessfully connected linker sequences will remain in the library. The impact on the subsequent on-board sequencing process is uncertain. The inventors found through experiments that these remaining probes will not affect the quality of sequencing data.

Therefore, in a first aspect, the present invention provides a method for constructing a capture library suitable for next-generation sequencing platforms, comprising the following steps:
(1) obtaining fragmented DNAs;
(2) connecting the fragmented DNAs to a Y-shaped linker to obtain a pre-library;
(3) hybridizing the pre-library with a hybridization probe to obtain a hybridization product;
(4) eluting the hybridization product to obtain a capture library;
wherein the step (4) comprises a step (4a) of performing an alkali denaturation on the hybridization product.

In some embodiments, the method is used to construct a next-generation sequencing capture library.

In some embodiments, the fragmented DNAs are selected from naturally occurring short-fragment DNAs or short-fragment DNAs obtained by artificial disruption of genomic DNAs.

In some other embodiments, the naturally occurring short-fragment DNAs are selected from peripheral blood free DNAs, tumor free DNAs or naturally degraded genomic DNAs; and the artificial disruption of genomic DNAs are achieved by a sonication, a mechanical disruption, or an enzymatic digestion.

In some embodiments, the fragmented DNAs are derived from a sample of the following group: blood, serum, plasma, joint fluid, semen, urine, sweat, saliva, stool, cerebrospinal fluid, ascites, pleural fluid, bile, or pancreatic fluid.

Preferably, the fragmented DNAs are 150 to 400 bp in length, preferably 180 to 230 bp.

In some embodiments, the method further comprises a step of performing end repair and/or end-addition of A to the fragmented DNAs after step (1). Preferably, the end repair and end-addition of A are performed in one reaction system. More preferably, the DNA fragmentation, end repair, and end-addition of A are performed in one reaction system.

In some embodiments, the Y-shaped linker in step (2) is a long Y-shaped linker comprising an amplification primer, an index tag sequence, a read 1/read 2 sequencing primer, and an index read sequencing primer.

In some embodiments, step (3) is carried out in a liquid phase system.

In some embodiments, in step (4) and after step (4a), the method further comprises:
step (4b): incubation;
step (4c): removal of magnetic beads; and
step (4d): neutralization.

In a preferred embodiment, the alkali reagent used for alkali denaturation in step (4a) is one or more selected from the group consisting of NaOH, KOH, lithium hydroxide, calcium hydroxide, magnesium hydroxide, ammonium hydroxide, lithium carbonate, sodium carbonate, potassium carbonate, sodium bicarbonate, potassium bicarbonate, ammonium bicarbonate, sodium methoxide, potassium methoxide, sodium ethoxide, potassium ethoxide, sodium tert-butoxide or potassium tert-butoxide. Further preferably, the alkali reagent is NaOH or KOH, and more preferably an aqueous solution thereof.

In a preferred embodiment, the concentration of the alkali reagent is between 0.05M and 1M, preferably between 0.1M and 0.5M, and more preferably 0.2M.

Preferably, in step (4a), an alkali reagent is added to the hybridization product to perform the alkali denaturation; and the pH value of the system after the addition of alkali reagent is between 11 and 14, further preferably between 12 and 14, and most preferably between 12 and 13.

Further preferably, the neutralization step is performed with a neutralizing reagent; preferably, the neutralizing reagent is selected from one or more of Tris-HCl, acetic acid, citrate buffer, phosphate buffer or acetate buffer. Preferably, the neutralizing reagent is selected from Tris-HCl.

In another aspect, the present application relates a kit for constructing a capture library comprising:
(a) a reagent for connecting a linker, comprising a Y-shaped linker;
(b) a reagent for hybridization; and
(c) a reagent for eluting a hybridization product.

In some embodiments, the Y-shaped linker is a long Y-linker comprising an amplification primer, an index tag sequence, a read 1/read 2 sequencing primer, and an index read sequencing primer.

In some embodiments, the kit further comprises a reagent for performing end repair and/or end-addition of A.

In some embodiment, the reagent for hybridization comprises a hybridization buffer, a Cot-1 DNA, and a hybridization probe; preferably, the reagent for hybridization does not comprise a blocking sequence.

In some embodiments, the blocking sequence comprises sequences designed to be reverse complementary to the linker and/or the tag sequence.

In some embodiments, the reagent for eluting the hybridization product comprises a denaturant and a neutralizing agent. Preferably, the denaturant is an alkali denaturant.

In a preferred embodiment, the alkali denaturant is selected from one or more of NaOH, KOH, lithium hydroxide, calcium hydroxide, magnesium hydroxide, ammonium hydroxide, lithium carbonate, sodium carbonate, potassium carbonate, sodium bicarbonate, potassium bicarbonate, ammonium bicarbonate, sodium methoxide, potassium methoxide, sodium ethoxide, potassium ethoxide, sodium tert-butoxide or potassium tert-butoxide; preferably NaOH or KOH, more preferably an aqueous solution thereof. The neutralizing agent is selected from one or more of Tris-HCl, acetic acid, citrate buffer, phosphate buffer or acetate buffer; preferably Tris-HCl.

In another aspect, the present invention further comprises a capture library constructed according to the method described above, or a capture library constructed according to the kit described above. In a preferred embodiment, the capture library is used in a next-generation sequencing platform.

### The excellent technical effects of the present invention are:

(1) The requirement for initial DNA content is relatively low, even as low as 25ng, which greatly improves the utilization of rare samples and expands the application scope of the present invention. For example, the method and kit of the present invention can be applied to dried blood spots, oral swabs and other sample types that cannot meet the ordinary exon capture process due to a small amount of DNA extraction.
(2) The library construction process is simple. The method of the present invention completely achieves the entire process of PCR-free before and after capture, thus simplifying and shortening the experimental process.
(3) Since the present invention does not involve a PCR amplification, it saves the cost of library construction related to amplification. The low redundancy advantage of the PCR-Free library itself increases the effective data utilization, thereby avoiding the waste of data volume and realizing the reduction of sequencing cost.
(4) PCR-Free library can perfectly avoid amplification errors and biases, display the true appearance of DNA sequences, and effectively improve mutation detection performance.

The invention will be described in more detail by way of examples with the accompanying drawings. It should be understood by those skilled in the art that the drawings and their examples are for illustrative purposes only and are not to be construed as limiting the invention in any way. The embodiments and features of the embodiments in the present application can be combined with each other without contradiction.

### BRIEF DESCRIPTION OF THE DRAWINGS

**FIG. 1****:** a flow diagram of the traditional exon capture process.
**FIG. 2****:** a flow diagram of the exon capture process optimized by the inventor (described in patent application CN110409001A).
**FIG. 3****:** a flow diagram of the PCR-Free library construction method of the entire library construction process of the present invention.
**FIG. 4****:** a structural diagram of the Y-shaped linker.
**FIG. 5****:** a microscopic diagram of the hybridization capture system.
**FIG. 6****:** measurement results of library concentrations.

### DETAILED DESCRIPTION

### Methods for constructing a capture library

In a first aspect, the present invention provides a method of constructing a capture library.

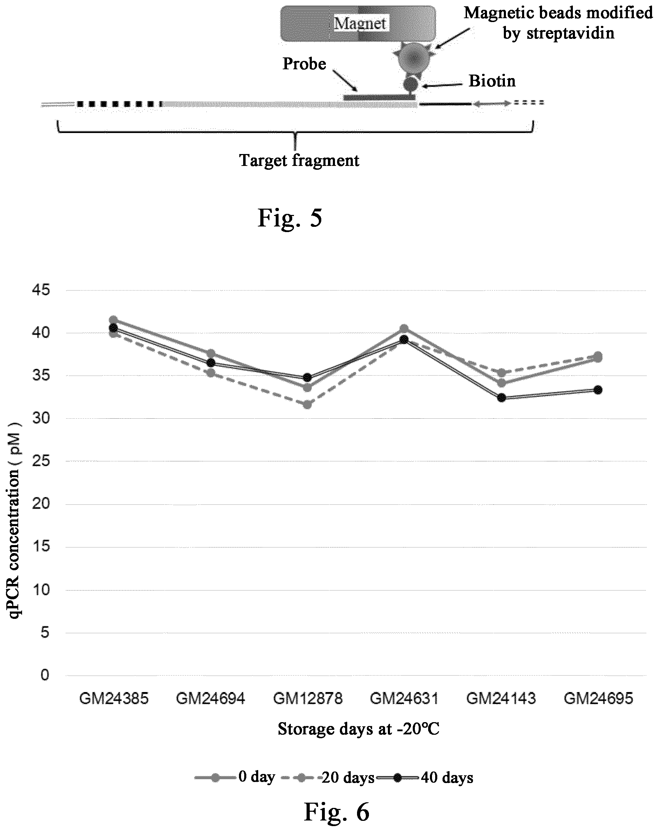

At the end of the traditional capture library construction, the hybridization product is often PCR amplified. On the one hand, the fragments captured by the probe are copied into the liquid phase system through the amplification (the covalent binding of the avidin on the streptavidin magnetic beads to the biotin on the probe is relatively strong. Meanwhile, the probe and the target fragment also form a double-stranded structure through complementary base pairing. See **FIG. 5****:** the microscopic diagram of the hybridization capture system). On the other hand, the final library output is improved to meet the on-board sequencing requirements. The introduction of post-hybridization PCR not only increases experimental costs and operational complexity, but also brings about various disadvantages of the PCR amplification (amplification errors and biases, which are not conducive to mutation detection. High redundancy increases sequencing costs).

In order to overcome the above disadvantages and achieve the true PCR-free entire library construction process, the inventors tried to develop a method that can directly detach the target fragment (library captured by the probe) from the streptavidin magnetic beads into the liquid phase system (after hybridization reaction).

The first method that comes to mind was to use free biotin to compete with the biotin on the probe, thus a double strand was formed by a part of the detached probe and the target fragment. However, after testing, it was found that the binding of avidin to biotin on the probe was too tight, and the concentration of the library obtained in the experiment was extremely small.

Subsequently, Deep Vent DNA polymerase with strand displacement activity was used to try to displace the target fragments from the probe, so as to obtain a double-stranded library with a higher stability. Through testing, it was found that due to the need to add enzymes and single-ended primers during the strand displacement step, the purification step of XP magnetic beads needs to be introduced after the displacement reaction, which result in the waste of the total amount of the final library. The resulting library cannot meet the requirements of multiple on-board sequencing. It does not bring simplification and cost savings to the overall experimental process, either.

Finally, a denaturation method (breakage of hydrogen bonds of double-stranded base pairs) was used to unravel the double strands formed by the probe and the target fragments. Currently, common denaturation methods include a high temperature and an extreme pH. After gradient testing, it was found that the process to denature the double strands through the means of a high temperature was difficult to operate and difficult to control. The concentration and stability of the library obtained in the experiment were poor.

After excluding the above experimental conditions, the experimental process of alkali denaturation was finally selected in the present invention. By adding alkali reagents, strong alkaline conditions were created for double-stranded unraveling. Through incubation and vortexing, the double-stranded unraveling was promoted. At the end of the experimental process, the alkalinity of the system was reduced by neutralization, which also facilitates the preservation of single-stranded library. The final library meets the requirements of multiple on-board sequencing and long-term storage.

Accordingly, the present application provides a method for constructing a capture library with high detection performance,
(1) obtaining fragmented DNAs;
(2) connecting the fragmented DNAs to a Y-shaped linker to obtain a pre-library;
(3) hybridizing the pre-library with a hybridization probe to obtain a hybridization product;
(4) eluting the hybridization product to obtain a capture library;
wherein the step (4) comprises a step (4a) of performing an alkali denaturation on the hybridization product.

In some embodiments, the method is used to construct a next-generation sequencing capture library.

In some embodiments, the fragmented DNAs refer to naturally occurring short-fragment DNAs or short-fragment DNAs obtained by artificial disruption of genomic DNAs.

In some embodiments, the fragmented DNAs may be derived from a sample such as blood, serum, plasma, joint fluid, semen, urine, sweat, saliva, stool, cerebrospinal fluid, ascites, pleural fluid, bile, or pancreatic fluid.

In a preferred embodiment, the naturally occurring short-fragment DNAs are peripheral blood free DNAs, tumor free DNAs or naturally degraded genomic DNAs.

In some other embodiments, genomic DNAs may derived from various sources, such as oral swabs, amniotic fluid, dried blood films, tissues, peripheral blood, and the like. The person skilled in the art is aware of a method for disrupting genomic DNAs, e.g., by a sonication, a mechanical disruption or an enzymatic digestion, and the like. Since the sonication and mechanical disruption lose relatively much DNAs, it is preferable for DNA fragmentation with the enzymatic digestion in the presence of a little initial amount of DNAs (e.g., as low as 50 ng).

In some embodiments, the fragmented DNAs are 150 to 400 bp in length, preferably 180 to 230 bp.

In some embodiments, the method of the invention further comprises the step (1') of end repair and/or end-addition of A of the fragmented DNAs prior to being connected to the Y-shaped linker (i.e., step 2). In this embodiment, the DNAs can be end-repaired using any enzyme known to those skilled in the art suitable for end-repair, such as T4 DNA polymerase, Klenow enzyme, or mixture thereof. In this embodiment, the DNAs can be end-added with A using any suitable enzyme for end-addition of A known to those skilled in the art. Examples of such enzymes include, but not limited to, Taq enzyme, klenow ex-enzyme, or mixture thereof. In this embodiment, end repair and end-addition of A may be carried out in two reaction systems, i.e., end-addition of A may be performed after end-repair followed by purification. Alternatively, and preferably, the steps of end-repair and end-addition of A are performed in one reaction system, i.e., end-repair and end-addition of A are made simultaneously, followed by purification of the nucleic acid. Alternatively, and more preferably, the steps of DNA fragmentation, end repair, and end-addition of A are performed in one reaction prior to ligation of the linker.

The above-mentioned method not only simplifies the procedure and saves cost, but also reduces contamination between samples.

In some embodiments, the incubation time and temperature used for end-filling and end-addition of A can be determined by those skilled in the art according to routine technique in line with specific demand.

In some embodiments, step (2) may be performed with any enzyme suitable for the ligation of the linker known to those skilled in the art. Examples of such enzymes include, but not limited to, T4 DNA ligase, T7 DNA ligase, or mixtures thereof. Conditions for carrying out the ligation reaction are well known to those skilled in the art.

In the context of the present invention, the "Y-shaped linker" refers to a linker formed by two strands that are not completely complementary, wherein one end of the linker forms a double strand due to complementarity between bases of the two strands, and the other end does not form a double strand due to incomplete complementarity between bases of the two strands. The present invention is suitable for ordinary Y-shaped linkers (True Seq linkers), as shown in **FIG. 4****:** the structural diagram of the Y-shaped linker.

An ordinary Y-shaped linker mainly comprises amplification primer sequences (P5/P7), index tag sequence, read 1/read 2 sequencing primer sequence and index read sequencing primer sequence, wherein the sequences of read 1/read 2 sequencing primer sequence and index read sequencing primer are not completely complementary to form a partial double strand.

For example, the Y-shaped linker available in the present invention comprises sequences of two strands as follows:
5'-AATGATACGGCGACCACCGAGATCTACACTCTTTCCCTACACGACGCTCTTCC GATCT-3'; and
5'-GATCGGAAGAGCACACGTCTGAACTCCAGTCAC{index}ATCTCGTATGCCGTC TTCTGCTTG-3' (having a phosphorylation modification at 5' end).

The complementary portions of two strands are underlined.

Methods for phosphorylation modification of oligonucleotide are well known to those skilled in the art. For example, oligonucleotide can be phosphorylated at 5' end by polynucleotide kinases, or phosphate group can be added directly to 5' end when primer is synthesized.

In some embodiments, step (3) of the method of the present invention is carried out in a liquid phase hybridization system.

Through previous technical optimization in this laboratory, a better capture efficiency can be achieved in the case of using the Y-shaped linker without PCR pre-amplification for preparation of pre-library in hybridization system without addition of any blocking sequence, thereby achieving the elimination of the amplification and library construction process for the pre-library prior to the hybridization, optimizing the analysis performance and reducing experimental costs.

Thus, in some embodiments, a system for hybridization includes a hybridization buffer, Cot-1 DNAs, and a hybridization probe, but no blocking sequence. The conditions for hybridization, such as hybridization temperature, hybridization time and the like, can be adjusted by one skilled in the art according to actual demand. The general principle for designing and preparing hybridization probe is also well known to those skilled in the art.

In some embodiments, step (4) of the method of the present invention further comprises a step of eluting the hybridization product to obtain a final capture library. Specifically, the step (4) comprises:
step (4a): an alkali denaturation performed on the hybridization product;
step (4b): incubation;
step (4c): removal of magnetic beads; and
step (4d): neutralization.

In a preferred embodiment, during the alkali denaturation process, the alkali denaturants that can be used and their concentrations are well known and selected by those skilled in the art. Preferably, for example, the reagent used in the alkali denaturation is an inorganic alkali or an organic alkali. Further, the inorganic alkali is selected from one or more of NaOH, KOH, lithium hydroxide, calcium hydroxide, magnesium hydroxide, ammonium hydroxide, lithium carbonate, sodium carbonate, potassium carbonate, sodium bicarbonate, potassium bicarbonate, ammonium bicarbonate; and the organic alkali is selected from one or more of sodium methoxide, potassium methoxide, sodium ethoxide, potassium ethoxide, sodium tert-butoxide or potassium tert-butoxide.

In a further preferred embodiment, the alkali denaturant is selected from the inorganic alkali, preferably selected from NaOH or KOH; more preferably an aqueous solution of the above-mentioned reagents. Further, the concentration of the alkali denaturant is between 0.05M and 1M, preferably between 0.1M and 0.5M, more preferably 0.2M.

In a preferred embodiment, in the alkali denaturation step, an alkali reagent is added to adjust the pH value of the system to denature the hybridization product. The pH adjustment range of the system is well known to those skilled in the art. For example, preferably, the system can be adjusted to a pH value of between 11 and 14, more preferably between 12 and 14, most preferably between 12 and 13.

In the context of the present invention, the term "pH value" comprises the conventional usage of the term, such as the logarithm of the reciprocal of the hydrogen ion concentration. The "pH value" herein also comprises an observed pH value, i.e. the pH value measured by contacting a medium with a conventional pH meter of a known type, suitably calibrated by a known method. Typically, the medium used in the measurement method of the present invention may contain some water. As used herein, the "pH value" of the solution generally refers to, for example, the pH value at room temperature (25°C).

In some embodiments, the method further comprises the following process after the addition of the alkaline denaturant: incubating the reaction system; after the incubation, performing an adsorption to remove the magnetic beads, and finally adding a neutralizing reagent to reduce the alkalinity of the system. Preferably, for example, the incubation is performed at room temperature. Those skilled in the art can select any suitable reagent for use to neutralize the above-mentioned alkaline denaturants and determine the concentration range thereof, such as Tris-HCl, acetic acid, citrate buffer, phosphate buffer or acetate buffer and the like.

In a preferred embodiment, the neutralizing reagent is selected from Tris-HCl; the concentration range of Tris-HCl is preferably between 100nM and 10mM, more preferably between 100 and 500nM, and most preferably 400nM.

### Kit

In a second aspect, the present invention further provides a kit for constructing a capture library comprising:
(a) a reagent for connecting a linker, comprising a Y-shaped linker;
(b) a reagent for hybridization; and
(c) a reagent for eluting a hybridization product.

In some embodiments, the Y-shaped linker is a long Y-linker comprising an amplification primer, an index tag sequence, a read 1/read 2 sequencing primer, and an index read sequencing primer.

In some embodiments, the kit further comprises a reagent for performing end repair and/or end-addition of A.

In some embodiments, the reagent for hybridization comprises a hybridization buffer, a Cot-1 DNA, and a hybridization probe; preferably, the reagent for hybridization does not comprise a blocking sequence, wherein the blocking sequence comprises sequences designed to be reverse complementary to the linker and/or the tag sequence.

In some embodiments, the reagent for eluting the hybridization product comprises a denaturant and a neutralizing agent; preferably, the denaturant is an alkali denaturant.

In a preferred embodiment, the alkali denaturant is selected from one or more of NaOH, KOH, lithium hydroxide, calcium hydroxide, magnesium hydroxide, ammonium hydroxide, lithium carbonate, sodium carbonate, potassium carbonate, sodium bicarbonate, potassium bicarbonate, ammonium bicarbonate, sodium methoxide, potassium methoxide, sodium ethoxide, potassium ethoxide, sodium tert-butoxide or potassium tert-butoxide. The neutralizing agent is selected from one or more of Tris-HCl, acetic acid, citrate buffer, phosphate buffer or acetate buffer.

In a further preferred embodiment, the alkali denaturant is selected from NaOH or KOH, more preferably the aqueous solution thereof. The neutralizing agent is selected from Tris-HCl. Specifically, the concentration of the aqueous solution of NaOH or KOH is between 0.1M and 0.5M, more preferably 0.2M. The concentration of Tris-HCl is preferably between 100nM and 500nM, more preferably 400nM.

In some embodiments, the capture library prepared according to the method of the present invention or the capture library prepared by the kit of the present invention is suitable for a variety of next-generation sequencing platforms, including but not limited to, for example, Roche/454 FLX, Illumina/Hiseq, Miseq, NextSeq and Life Technologies/SOLID system, PGM, Proton, and the like.

### EXAMPLE

**Example 1:** A library was constructed using different input amounts of templates. On-board sequencing was performed. Data was analyzed. The sequencing data quality control as well as performance analysis results of different input amounts were compared.

In this example, genomic DNAs of a standard cell line GM24385 were used as a template. The capture libraries with initial DNA input amounts of 25, 50, 75, 100, 150, and 200ng were constructed according to the method of the present invention. The libraries were sequenced on the NextSeq CN500 sequencing platform (150 bp paired-end sequencing). Bioinformatics was used to analyze the sequencing results. The library qualities of different template input amounts were analyzed.

### Step 1: Obtaining fragmented DNAs, end repair and end-addition of A

According to the manufacturer's instructions, 5×WGS Fragmentation Mix kit (Enzymatics, Cat. No. Y9410L) and the matched 10×Fragmentation Buffer kit (Enzymatics, B0330) were used to prepare the reaction system shown in Table 1 to complete the fragmentation, end repair and end-addition of A in one step.

**Table 1: Components and amounts thereof used in the reaction of step 1**

| **Components** | **Amounts used in a single reaction (µl)** |
|---|---|
| **Genomic DNAs** | X (corresponding volume of 25, 50, 75, 100, 150, and 200ng) |
| **5×WGS Fragmentation Mix** | 5 |
| **10× Fragmentation Buffer** | 2.5 |
| **Enzyme-free water** | 17.5-X |
| **Total volume** | 25 |

The reaction system was reacted according to the procedure in **Table 2.**

**Table 2: Reaction procedure**

| **Reaction procedure** | **Temperature** | **Time/min** |
|---|---|---|
| 1 | 4°C | 1 |
| 2 | 32°C | 16 |
| 3 | 65°C | 30 |
| 4 | 4°C | Hold |

### Step 2: connecting the linkers

The library linker constructed in the present invention was an ordinary Y-shaped linker (True Seq linker). The WGS Ligase kit (Enzymatics, Cat. No. L6030-600000) was used to prepare the ligation system shown in **Table 3** using the reaction product of step 1. The ligation system was incubated at 20°C for 15 minutes and then held at 4°C.

**Table 3: Components and amounts thereof used in the ligation of step 2**

| **Components** | **Amounts used in a single reaction (µl)** |
|---|---|
| **Reaction system of step 1** | 25 |
| **Y-shaped linker** | 5 |
| **T4 DNA Ligase (High Concentration)** | 5 |
| **5X Rapid Ligation Buffer** | 10 |
| **Enzyme-free water** | 5 |
| **Total volume** | 50 |

At the end of the ligation reaction, the ligation product was purified using the Beckman Agencourt AMPure XP kit (Beckman, Cat. No. A63882).

### Step 3: Capturing hybridization

A xGen Lockdown Reagents kit (IDT, Cat. No. 1072281) and Streptavidin Dynbeads M270 magnetic beads (Thermo Fisher Scientific, Cat. No. 35302) were used. 14.5µl hybridization reagent (9.5µl xGen 2× hybridization buffer, 3µl xGen hybridization buffer enhancer and 2µl Cot-1 DNA) was added to the purified product in step 2, mixed thoroughly and incubated at room temperature for 10 minutes. At the end of the incubation, 12.75µl of the supernatant was transferred into a new low-adsorption 0.2 mL centrifuge tube, followed by the addition of 4.25µl hybridization probe. At the end of incubation, centrifugation was performed immediately after sufficient mixing. Capturing hybridization was performed according to the procedure described in **Table 4** below.

**Table 4: Capturing hybridization procedure**

| **Reaction procedure** | **Temperature** | **Time** | **Cycle** |
|---|---|---|---|
| 1 | 95°C | 30s | |
| 2 | 65°C | 60s | 60 cycles |
| 3 | 37°C | 3s | |
| 4 | 65°C | 16h | |
| 5 | 65°C | Hold | |

After hybridization, the hybridization product (i.e., the magnetic beads bound to the target sequence) was washed and purified using the xGen Lockdown Reagents kit (IDT, Cat. No. 1072281) according to the manufacturer's instructions.

### Step 4: Eluting the target fragment

3.5µL NaOH solution (0.2 M) and 15µL enzyme-free water were added to the purified hybridization product (with beads) in step 3, mixed thoroughly and incubated at room temperature for 10 minutes. The product was vortexed alternately during the period. At the end of the incubation, the product was placed on a magnetic stand for 5 minutes, and 18µL of the supernatant was transfered into a low-adsorption 0.2 mL centrifuge tube that has been added with 4µL Tris-Hcl (400 nM). The final capture library was obtained after vortexing and mixing.

The capture library was quantified by qPCR, and then the library was sequenced (150 bp paired-end sequencing) using the NextSeq CN500 sequencing platform according to the standard operating procedures of the sequencer. 2.5G data was measured for each sample. The sequencing results, including basic quality control and standard cell line SNV&INDEL performance analysis, are shown in **Table 5.**

**Table 5: Sequencing results of Example 1**

| **Template input amount (ng)** | **qPCR concentration (pM)** | **Mapping rate** | **Duplication rate** | **Uniformity** | **Average sequencing depth** | **Capture rate** | **Key gene 20× coverage** | **SNP** | | **INDEL** | |
|---|---|---|---|---|---|---|---|---|---|---|---|
| | | | | | | | | **Sensitivity%** | **Accuracy%** | **Sensitivity%** | **Accuracy%** |
| 25 | 15.4205 | 93.49% | 1.98% | 27.57 | 61.94 | 37.77% | 97.80% | 99.30% | 99.95% | 92.61% | 97.77% |
| 50 | 26.2985 | 93.48% | 1.83% | 27.41 | 60.94 | 37.12% | 97.73% | 99.47% | 99.93% | 93.13% | 97.78% |
| 75 | 32.19 | 93.60% | 1.77% | 26.65 | 62.53 | 38.06% | 97.86% | 99.51% | 99.97% | 93.14% | 97.61% |
| 100 | 38.3225 | 94.01% | 1.58% | 27 | 68.91 | 41.50% | 98.32% | 99.55% | 99.90% | 93.84% | 98.34% |
| 150 | 32.529 | 93.71% | 1.66% | 26.36 | 64.36 | 39.07% | 98.05% | 99.54% | 99.93% | 94.37% | 97.63% |
| 200 | 32.4535 | 93.52% | 1.65% | 27.43 | 61.76 | 37.60% | 97.90% | 99.43% | 99.92% | 93.14% | 98.15% |

It can be seen from the above table that under the same data volume and within the input range of 25ng-200ng DNA template, the capture library constructed according to the method of the present invention has no significant difference in basic quality control and performance analysis, except for qPCR concentration. This demonstrates that according to the method of the present invention, samples with an initial DNA content as low as 25ng can be used, and the prepared capture library fully meets the requirements of on-board sequencing and subsequent data analysis. The "uniformity" in the table is IQR (interquartile range, also known as quartile difference), which herein refers to the difference in sequence coverage between 25% and 75% of the density depth distribution. This value is a measure of the variability of the results, which reflects the uneven coverage across the data set. A high IQR indicates a high variability in genome coverage, whereas a low IQR reflects a more uniform sequence coverage.

**Comparative Example 1:** At the end of step 3 in the process of Example 1, step (4) in Example 1 was replaced with a PCR amplification step (12 cycles, see Table 7 for details). Different template input amounts were used to construct library. On-board sequencing was performed. Data was analyzed. The sequencing data qualities as well as performance analysis results of different input amounts (50ng, 75ng and 100ng respectively) were compared. The results above were then compared with the analysis results of the corresponding same template input amounts in Example 1.

Genomic DNAs of a standard cell line GM24385 were used as a template in this comparative example. At the end of step 3 in the process of Example 1, the purified hybridization product (with beads) was PCR amplified to prepare the final library. The capture libraries with an initial DNA input amounts of 50ng, 75ng, and 100ng were constructed respectively. The libraries were sequenced on the NEXTSEQ CN500 sequencing platform (150 bp paired-end sequencing). Bioinformatics was used to analyze the sequencing results. The library qualities of different template input amounts were analyzed.

Specifically, according to the manufacturer's instructions, 2×KAPA HiFi Hot Start Ready Mix kit (KAPA, Cat. No. KK2602) was used to prepare the amplification system shown in **Table 6.** The sequences of the pre-amplification primers were as follows:
P5 primer: 5'-AATGATACGGCGACCACCGA-3';
P7 primer: 5'-CAAGCAGAAGACGGCATACGA-3'.

**Table 6: Components and amounts thereof used in the amplification system**

| **Components** | **Amounts used in a single reaction (µl)** |
|---|---|
| **hybridization product (with beads) & Nuclease-free water** | 23 |
| **2×KAPA HiFi Hot Start Ready Mix** | 25 |
| **25µM P5+P7 primer mixture** | 2 |
| **Total volume** | 50 |

PCR was performed according to the procedure in **Table 7** below.

**Table 7: PCR procedure**

| **PCR procedure** | **Temperature** | **Time** | **Cycle** |
|---|---|---|---|
| 1 | 98°C | 45s | |
| 2 | 98°C | 15s | 12 cycles |
| 3 | 65°C | 30s | |
| 4 | 72°C | 30s | |
| 5 | 72°C | 60s | |
| 6 | 4°C | Hold | |

At the end of the PCR procedure, a Beckman Agencourt AMPure XP kit (Beckman, Cat. No. A63882) was used for purification to obtain the final capture library.

The capture libraries prepared according to Comparative Example 1 was quantified by qPCR. The libraries were then sequenced (150 bp paired-end sequencing) using the NextSeq CN500 sequencing platform according to the standard operating procedures of the sequencer. 2.5G data was measured for each sample. The sequencing results were shown in **Table 8.**

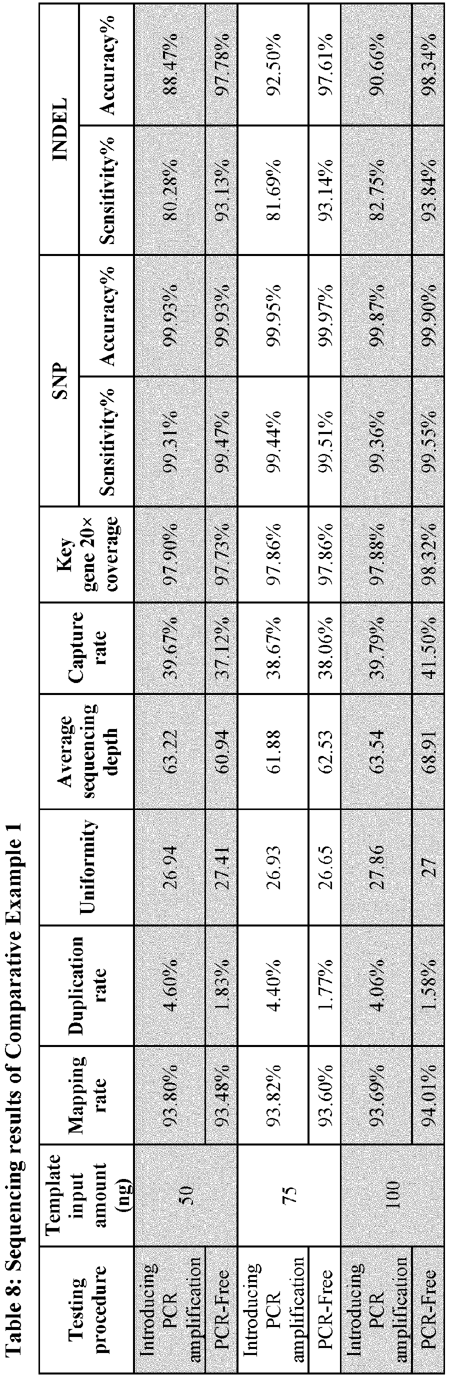

As can be seen from the above table, under the same data volume, the data fluctuations between the libraries constructed by the present invention (PCR-Free in **Table 8**) and the PCR amplification-introduced libraries are not obvious under different template input amounts. The duplication rates of the PCR-Free libraries were significantly lower than those of the PCR-introduced process (the average duplication rate was 1.73% when the template amounts were 50/75/100 ng and the average duplication rate of the PCR-introduced process was 4.35%), resulting in effective utilization of the on-board data. Moreover, the mutation detection performance of the library constructed by the present invention improved. Notably, the detection performance of INDEL significantly improved (the average sensitivity and accuracy were 93.37% and 97.91% respectively when the template amounts were 50, 75, and 100 ng, whereas the average sensitivity and accuracy were 81.57% and 90.63% respectively when the template amounts of the libraries constructed by the PCR-introduced process were 50, 75, and 100 ng).

**Comparative Example 2:** At the end of step 3 in the process of Example 1, step (4) in Example 1 was replaced with the elution of the target fragment using a saturated solution of free biotin (also known as D-biotin). After the library was constructed by 100 ng of template input amount, a qPCR concentration was quantified. The results above were compared with the library concentration results of the corresponding same template input amount in Example 1.

Genomic DNAs of a standard cell line GM24385 were used as a template in this comparative example. At the end of step 3 in the process of Example 1, a saturated solution of free avidin was added to the purified hybridization product (with beads) and incubated with heating, eventually achieving the purpose to elute the final library.

Specifically, free biotin (Thermo Fisher, Cat. No. B1595) was dissolved in enzyme-free water to prepare a saturated solution (approximately 0.2 mg/ml). 22.5µL free biotin saturated solution was added to the purified hybridization product (with beads) in step 3, mixed thoroughly and incubated at 37°C for 30 minutes. At the end of the incubation, the product was placed on a magnetic stand for 5 minutes, and 22µL of the supernatant was transferred into a 0.2 mL low-adsorption centrifuge tube, vortexed and mixed to obtain the final capture library.

The capture library prepared in Comparative Example 2 was subjected to qPCR quantification, and the library concentrations were shown in **Table 9.**

**Table 9: qPCR results**

| **Elution method for PCR-Free final library** | **QC result (pM)** |
|---|---|
| Alkaline denaturation | 38.3225 |
| free biotin displacement | 1.2745 |

As can be seen from the table above, when the input amounts of template DNA were the same, the concentration of the library obtained by free biotin displacement method was too low to meet the on-board sequencing requirements.

**Comparative Example 3:** At the end of step 3 in the process of Example 1, step (4) in Example 1 was replaced with a strand displacement reaction of the target fragment using Deep Vent DNA polymerase. After the library was constructed by 100 ng of template input amount, a qPCR concentration was quantified. The results above were compared with the library concentration results of the corresponding same template input amount in Example 1.

Genomic DNAs of a standard cell line GM24385 were used as a template in this comparative example. At the end of step 3 in the process of Example 1, the purified hybridization product (with beads) was subjected to a strand displacement reaction to prepare a final library.

Specifically, according to the manufacturer's instructions, Deep Vent^{®} DNA Polymerase (NEB, Cat. No. M0258S) was used to prepare the strand displacement system shown in **Table 10.** The sequence of the strand displacement primer was as follows:
P7 primer: 5'-CAAGCAGAAGACGGCATACGA-3'.

**Table 10: Components and amounts thereof used in the strand displacement system**

| **Components** | **Amounts used in a single reaction (µl)** |
|---|---|
| **hybridization product (with beads) & Nuclease-free water** | 19.5 |
| **ThermoPol Reaction Buffer (10X)** | 2.5 |
| **Deoxynucleotide (dNTP) Solution Mix (10 mM)** | 1 |
| **7' Primer (10 µM)** | 0.5 |
| **Deep Vent DNA Polymerase** | 0.25 |
| **MgSO₄** | 1.25 |
| **Total volume** | 25 |

Reaction was performed according to the procedure in **Table 11** below.

**Table 11: Reaction procedure**

| **Reaction procedure** | **Temperature** | **Time** |
|---|---|---|
| 1 | 95°C | 30s |
| 2 | 65°C | 30s |
| 3 | 72°C | 48s |
| 4 | 4°C | Hold |

At the end of the PCR procedure, Beckman Agencourt AMPure XP kit (Beckman, Cat. No. A63882) was used for purification to obtain the final capture library.

The capture libraries prepared according to Comparative Example 3 was quantified by qPCR. The library concentration was shown in Table 12.

**Table 12: qPCR results**

| **Elution method for PCR-Free final library** | **QC result (pM)** |
|---|---|
| Alkaline denaturation | 38.3225 |
| Strand displacement | 12.1325 |

As can be seen from the table above, when the input amounts of template DNAs were the same, the concentration of the library obtained by the strand displacement method was approximately 31.66% of that obtained by the alkaline denaturation method. The library concentrations varied greatly and did not meet the requirements for multiple on-board sequencing.

**Comparative Example 4:** At the end of step 3 in the process of Example 1, step (4) in Example 1 was replaced with a high-temperature thermal denaturation method to elute the target fragment under different incubation times (2 minutes, and 5 minutes). After the library was constructed by 100 ng of template input amount, a qPCR concentration was quantified. The results above were compared with the library concentration results of the corresponding same template input amount in Example 1.

Genomic DNAs of a standard cell line GM24385 were used as a template in this comparative example. At the end of step 3 in the process of Example 1, 22.5µL enzyme-free water was added to the purified hybridization product (with beads). After the double strands of DNAs were unwound and denatured under high temperature conditions, the magnetic beads were quickly removed to ultimately achieve the purpose to elute the final library.

Specifically, 22.5µL enzyme-free water was added to the purified hybridization product (with beads) in step 3, mixed thoroughly and incubated at 98°C for 2 minutes and 5 minutes respectively. At the end of the incubation, the product was placed on a magnetic stand for 1 minute to adsorb. 22µL of the supernatant was then transferred into a 0.2 mL low-adsorption centrifuge tube, vortexed and mixed to obtain the final capture library.

The capture library prepared according to Comparative Example 4 was subjected to qPCR quantification, and the library concentrations were shown in **Table 13.**

**Table 13: qPCR results**

| **Elution method for PCR-Free final library** | **QC result (pM)** |
|---|---|
| Alkaline denaturation | 38.3225 |
| Thermal denaturation for 2min | 0.054 |
| Thermal denaturation for 5min | 0.027 |

As can be seen from the table above, when the input amounts of template DNAs were the same, the concentration of the library obtained by thermal denaturation method was too low to meet the on-board sequencing requirements.

**Example 2:** The libraries constructed with the initial DNA input amount of 100 ng in Example 1 and the libraries constructed with the same template DNA input amount in PCR amplification-introduced process in Comparative Example 1 were subjected to on-board sequencing. The data volume of 2, 2.5, 3, 3.5, 4, 4.5, and 5G were intercepted respectively. Data analysis was performed. The sequencing data quality control and performance analysis results of two different library construction processes under different sequencing depths were compared. The analysis results were shown in **Tables 14(a) and 14(b)** respectively.

**Table 14(a): Sequencing data analysis results of the method in Example 1**

| **Testing procedure** | **Intercepted data volume (G)** | **Mapping rate** | **Duplication rate** | **Uniformity** | **Average sequencing depth** | **Capture rate** | **Key gene 20× coverage** | **SNP** | | **INDEL** | |
|---|---|---|---|---|---|---|---|---|---|---|---|
| | | | | | | | | **Sensitivity%** | **Accuracy%** | **Sensitivity%** | **Accuracy%** |
| PCR-Free | 2 | 94.02% | 1.09% | 27.67 | 55.46 | 41.52% | 97.29% | 99.36% | 99.86% | 92.79% | 97.60% |
| | 2.5 | 94.03% | 1.32% | 26.4 | 69.09 | 41.49% | 98.32% | 99.50% | 99.92% | 94.37% | 97.81% |
| | 3 | 94.02% | 1.55% | 26.01 | 82.63 | 41.47% | 98.68% | 99.60% | 99.93% | 95.25% | 97.83% |
| | 3.5 | 94.03% | 1.77% | 25.74 | 95.9 | 41.40% | 98.83% | 99.57% | 99.96% | 95.43% | 97.66% |
| | 4 | 94.02% | 1.98% | 25.22 | 109.2 | 41.35% | 98.91% | 99.60% | 99.96% | 95.42% | 97.83% |
| | 4.5 | 94.02% | 2.19% | 24.82 | 122.26 | 41.29% | 98.95% | 99.64% | 99.98% | 95.60% | 98.20% |
| | 5 | 94.02% | 2.39% | 24.78 | 135.24 | 41.23% | 98.98% | 99.64% | 99.98% | 96.13% | 97.33% |

**Table 14(b): Sequencing data analysis results of the method in Comparative Example 1**

| **Testing procedure** | **Intercepted data volume (G)** | **Mapping rate** | **Duplication rate** | **Uniformity** | **Average sequencing depth** | **Capture rate** | **Key gene 20× coverage** | **SNP** | | **INDEL** | |
|---|---|---|---|---|---|---|---|---|---|---|---|
| | | | | | | | | **Sensitivity%** | **Accuracy%** | **Sensitivity%** | **Accuracy%** |
| Introducing PCR amplification | 2 | 93.69% | 3.30% | 28.11 | 51.31 | 39.84% | 96.32% | 99.24% | 99.81% | 83.45% | 88.36% |
| | 2.5 | 93.69% | 4.06% | 27.86 | 63.54 | 39.79% | 97.88% | 99.36% | 99.87% | 82.75% | 90.66% |
| | 3 | 93.70% | 4.80% | 26.81 | 75.4 | 39.71% | 98.46% | 99.46% | 99.92% | 84.69% | 88.59% |
| | 3.5 | 93.70% | 5.52% | 26.94 | 86.94 | 39.61% | 98.72% | 99.54% | 99.93% | 85.39% | 88.05% |
| | 4 | 93.70% | 6.22% | 25.97 | 98.2 | 39.49% | 98.84% | 99.50% | 99.95% | 84.68% | 90.27% |
| | 4.5 | 93.70% | 6.91% | 25.57 | 109.28 | 39.41% | 98.90% | 99.55% | 99.93% | 85.92% | 88.25% |
| | 5 | 93.70% | 7.59% | 25.29 | 120.18 | 39.34% | 98.94% | 99.60% | 99.96% | 84.68% | 89.59% |

It can be seen from the two tables above that, as the sequencing data volume increases, the sequencing depth of the sample will change significantly. Changes in sequencing depth can optimize the detection performance of SNP and INDEL to a certain extent. Comparison of the data in the above two tables further demonstrates that the present invention has a greater advantage in the detection performance of INDEL, and the process of the present invention can obtain relatively stable detection performance under a data volume of 2.5G.

**Example 3:** Genomic DNAs of a standard cell line GM24385 were used as a template in this example to construct a capture library with an initial DNA input amount of 100 ng. The library construction method in this example was the same as that in Example 1, except that the incubation time at room temperature during the elution of the target fragment in step 4 was 5 minutes, 10 minutes and 15 minutes respectively. The library was sequenced (150 bp paired-end sequencing) on the NextSeq CN500 sequencing platform. Bioinformatics was used to analyze the sequencing results. The library quality at different incubation times was analyzed. The sequencing results were shown in **Table 15.**

**Table 15: Sequencing results of Example 3**

| **Incubation time (min)** | **qPCR concentration(pM)** | **Mapping rate** | **Duplication rate** | **Uniformity** | **Average sequencing depth** | **Capture rate** | **Key gene 20× coverage** | **SNP** | | **INDEL** | |
|---|---|---|---|---|---|---|---|---|---|---|---|
| | | | | | | | | **Sensitivity%** | **Accuracy%** | **Sensitivity%** | **Accuracy%** |
| 5 | 21.44 | 93.78% | 1.75% | 28.82 | 65.15 | 39.77% | 98.01% | 99.35% | 99.89% | 94.01% | 97.63% |
| 10 | 38.3225 | 94.01% | 1.58% | 27 | 68.91 | 41.50% | 98.32% | 99.55% | 99.90% | 93.84% | 98.34% |
| 15 | 44.4695 | 93.65% | 1.69% | 26.79 | 63.47 | 38.54% | 97.98% | 99.43% | 99.94% | 92.26% | 98.31% |

As can be seen from the table above, the library concentration increases with the extension of incubation time. All the libraries obtained under the three incubation conditions meet three-time on-board sequencing requirements. Under the same data volume, the basic quality control and SNP/INDEL detection performance of the libraries obtained under the three incubation conditions did not vary significantly. The mapping rate and key gene 20× coverage of the library under the 10-minute incubation time condition were higher. This incubation time condition was also more reasonable in terms of experimental operation time. The present invention thus recommends a process with an incubation time of 10 minutes at a room temperature.

**Example 4:** The library construction method in this example is the same as that in Example 1 (constructing a capture library with an initial DNA input amount of 100 ng), except that the DNA template was changed from the original standard cell line to a clinical positive sample. Capture libraries were prepared using oral swab DNAs, amniotic fluid DNAs, dried blood film DNAs, tissue DNAs, and peripheral blood DNAs respectively. The capture libraries were quantified by qPCR, and then sequenced (150 bp paired-end sequencing) using the NextSeq CN500 sequencing platform according to the standard operating procedures of the sequencer. 2.5G data was measured for each sample. The sequencing results are shown in **Table 16.**

**Table 16: Sequencing results of Example 4**

| **Sample type** | **QC result (pM)** | **Mapping rate** | **Duplication rate** | **Uniformity** | **Average sequencing depth** | **Capture rate** | **Key gene 20× coverage** |
|---|---|---|---|---|---|---|---|
| oral swab DNAs | 52.07 | 93.85% | 2.13% | 25.23 | 64.55 | 39.85% | 98.26% |
| amniotic fluid DNAs | 60.206 | 93.02% | 1.89% | 28.05 | 65.8 | 42.89% | 98.11% |
| dried blood film DNAs | 41.524 | 93.22% | 1.44% | 28.99 | 65.33 | 40.64% | 98.03% |
| tissue DNAs | 46.541 | 93.65% | 1.35% | 29.58 | 63.58 | 38.67% | 98.01% |
| peripheral blood DNAs | 43.362 | 94.76% | 1.35% | 23.84 | 71.94 | 42.65% | 98.45% |

For the 5 clinical positive samples used in the table above, the basic quality control of the libraries all met the standards and were correctly detected. Accordingly, the sequencing library construction method of the present invention can be applied to a variety of sample types, especially samples with less DNA content.

### Example 5: Study on the stability of library qPCR concentration

According to the method described in Example 1 (constructing a capture library with an initial DNA input of 100 ng), libraries were constructed using the genomic DNAs of six standard cell lines (GM24385, GM24694, GM12878, GM24631, GM24143 and GM24695) as templates. The library qPCR concentrations were measured at an interval of 0, 20, and 40 days (the libraries were stored at -20°C). The effect of storage time on library qPCR concentration was explored. The concentration measurement results are shown in **Table 17 and** **FIG. 6****.**

**Table 17: Effect of storage time on library qPCR concentration**

| **Standard cell line name** | **0 day** | **20 days** | **40 days** |
|---|---|---|---|
| GM24385 | 41.494 | 39.891 | 40.514 |
| GM24694 | 37.591 | 35.268 | 36.446 |
| GM12878 | 33.584 | 31.604 | 34.729 |
| GM24631 | 40.469 | 39.147 | 39.158 |
| GM24143 | 34.066 | 35.311 | 32.348 |
| GM24695 | 36.989 | 37.286 | 33.312 |

It can be seen from **Table 17 and** **FIG. 6** that the libraries constructed using the process of the present invention have no significant change in library concentration within 40 days of storage at -20°C and exhibit a good stability.

### Example 6: Study on the stability of detected library reloaded for on-board sequencing

Genomic DNA libraries of the six standard cell lines constructed in Example 5 were sequenced (150 bp paired-end sequencing) using the NextSeq CN500 sequencing platform according to the standard operating procedures of the sequencer. 2.5G data was measured for each sample. The libraries were stored at -20°C for 25 days and then reloaded (same data volume) for on-board sequencing to explore the impact of storage time on the stability of the detection of the library. The sequencing results are shown in

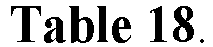

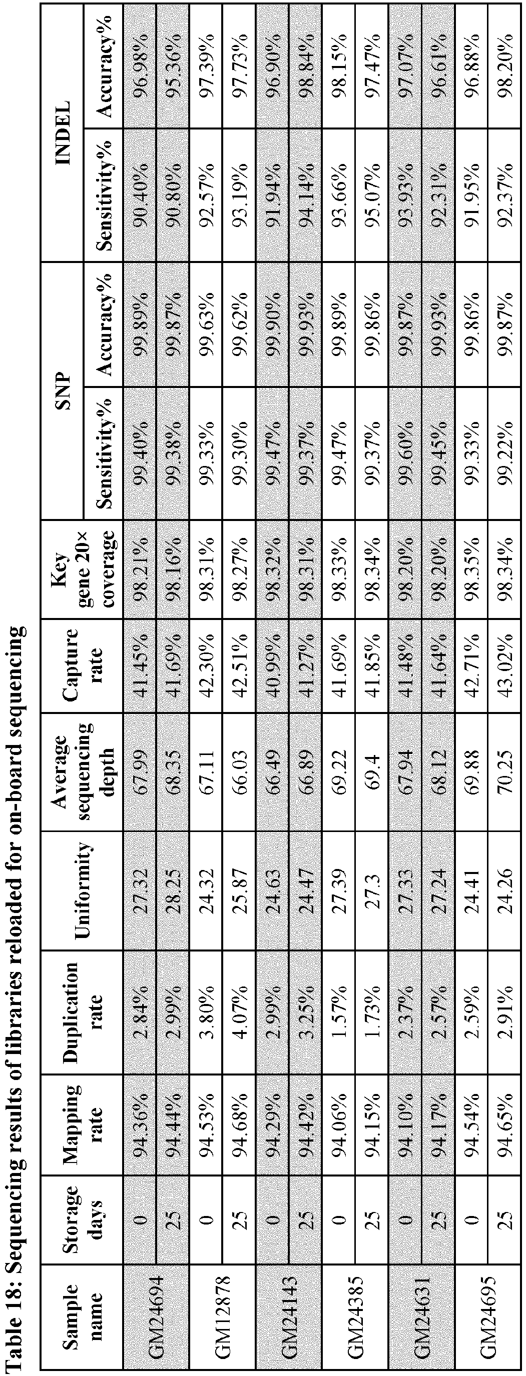

It can be seen from the table above that the quality of the libraries constructed using the process of the present invention did not change significantly within 25 days of storage at - 20°C. In combination with Example 5, it can be known that the libraries constructed by the process of the present invention are relatively stable and can be stored for a long time.

**It should be noted that the above are only preferred embodiments of the present invention and are not intended to limit the present invention. For those skilled in the art, the present invention may have various modifications and changes. Those skilled in the art understand that any modifications, equivalent substitutions, improvements and the like made within the spirit and principles of the present invention shall be included in the protection scope of the present invention.**

## Claims

1. A method for constructing a capture library suitable for next-generation sequencing platforms, comprising the following steps:
(1) obtaining fragmented DNAs;
(2) connecting the fragmented DNAs to a Y-shaped linker to obtain a pre-library;
(3) hybridizing the pre-library with a hybridization probe to obtain a hybridization product;
(4) eluting the hybridization product to obtain a capture library;
wherein the step (4) comprises a step (4a) of performing an alkali denaturation on the hybridization product.

2. The method of claim 1, wherein the fragmented DNAs are selected from naturally occurring short-fragment DNAs or short-fragment DNAs obtained by artificial disruption of genomic DNAs.

3. The method of claim 2, wherein the naturally occurring short-fragment DNAs are selected from peripheral blood free DNAs, tumor free DNAs or naturally degraded genomic DNAs; and the artificial disruption of genomic DNAs are achieved by a sonication, a mechanical disruption, or an enzymatic digestion.

4. The method of claim 1, wherein the fragmented DNAs are derived from a sample of the following group: blood, serum, plasma, joint fluid, semen, urine, sweat, saliva, stool, cerebrospinal fluid, ascites, pleural fluid, bile, or pancreatic fluid.

5. The method of any one of claims 1 to 4, wherein the fragmented DNAs are 150 to 400 bp in length.

6. The method of claim 1, further comprising a step of performing end repair and/or end-addition of A to the fragmented DNAs after step (1).

7. The method of claim 1, wherein the Y-shaped linker is a long Y-shaped linker comprising an amplification primer, an index tag sequence, a read 1/read 2 sequencing primer, and an index read sequencing primer.

8. The method of claim 1, wherein in step (4) and after step (4a), the method further comprises:
step (4b): incubation;
step (4c): removal of magnetic beads; and
step (4d): neutralization.

9. The method of claim 1, wherein the alkali reagent used for alkali denaturation in step (4a) is one or more selected from the group consisting of NaOH, KOH, lithium hydroxide, calcium hydroxide, magnesium hydroxide, ammonium hydroxide, lithium carbonate, sodium carbonate, potassium carbonate, sodium bicarbonate, potassium bicarbonate, ammonium bicarbonate, sodium methoxide, potassium methoxide, sodium ethoxide, potassium ethoxide, sodium tert-butoxide or potassium tert-butoxide.

10. The method of claim 9, wherein the concentration of the alkali reagent is between 0.05M and 1M.

11. The method of claim 1, wherein in step (4a), an alkali reagent is added to the hybridization product to perform the alkali denaturation; and wherein the pH value of the system after the addition of alkali reagent is between 11 and 14.

12. The method of claim 8, wherein in the step (4d), a neutralization is performed with a neutralizing reagent selected from one or more of Tris-HCl, acetic acid, citrate buffer, phosphate buffer or acetate buffer.

13. A kit for constructing a capture library comprising:
(a) a reagent for connecting a linker, comprising a Y-shaped linker;
(b) a reagent for hybridization; and
(c) a reagent for eluting a hybridization product.

14. The kit of claim 13, wherein the Y-shaped linker is a long Y-linker comprising an amplification primer, an index tag sequence, a read 1/read 2 sequencing primer, and an index read sequencing primer.

15. The kit of claim 13, wherein the kit further comprises a reagent for performing end repair and/or end-addition of A.

16. The kit of claim 13, wherein the reagent for hybridization comprises a hybridization buffer, a Cot-1 DNA, and a hybridization probe; and wherein the reagent for hybridization does not comprise a blocking sequence.

17. The kit of claim 16, wherein the blocking sequence comprises sequences designed to be reverse complementary to the linker and/or the tag sequence.

18. The kit of claim 13, wherein the reagent for eluting the hybridization product comprises a denaturant and a neutralizing agent; and wherein the denaturant is an alkali denaturant.

19. The kit of claim 18, wherein the alkali denaturant is selected from one or more of NaOH, KOH, lithium hydroxide, calcium hydroxide, magnesium hydroxide, ammonium hydroxide, lithium carbonate, sodium carbonate, potassium carbonate, sodium bicarbonate, potassium bicarbonate, ammonium bicarbonate, sodium methoxide, potassium methoxide, sodium ethoxide, potassium ethoxide, sodium tert-butoxide or potassium tert-butoxide; and the neutralizing agent is selected from one or more of Tris-HCl, acetic acid, citrate buffer, phosphate buffer or acetate buffer.

20. A capture library constructed by the method of any one of claims 1 to 12 or a capture library constructed by utilizing the kit of any one of claims 13 to 19.
